# EUROPEAN PATENT SPECIFICATION

(11) **EP 3 448 334 B1**
(45) Date of publication and mention of the grant of the patent: **25.02.2026**
(21) Application number: 17720487.2
(22) Date of filing: 28.04.2017
(51) Int. Cl.: A61F 7/02, F25D 3/08

(54) **COOLING ELEMENT, ITS MANUFACTURE AND ITS USE**
KÜHLELEMENT, DESSEN HERSTELLUNG UND DESSEN VERWENDUNG
ÉLÉMENT DE REFROIDISSEMENT, SA FABRICATION ET SON UTILISATION

(30) Priority: 29.04.2016 NL 1041849
(43) Date of publication of application: 06.03.2019
(73) Proprietor: Recycold Holding B.V., 5916 PJ Venlo (NL)
(72) Inventor: OOST, Lubbert Jelle, 8245JB Lelystad (NL)
(74) Representative: Bardehle Pagenberg Partnerschaft mbB Patentanwälte Rechtsanwälte
(86) International application number: PCT/EP2017/060306
(87) International publication number: WO 2017/186965

(56) References cited:
- EP-A2- 0 050 948
- WO-A1-2006/073341
- JP-A- 2000 255 589
- JP-A- 2001 171 688
- JP-A- 2009 072 404
- JP-A- 2010 240 193
- JP-A- 2012 006 659
- JP-A- H06 345 102
- US-A- 2 203 591
- US-A- 3 871 376
- MARWIN DEKKERS: "#dutchtopdesign | Facebook", 25 April 2016 (2016-04-25), XP055379451, Retrieved from the Internet <URL:https://www.facebook.com/hashtag/dutchtopdesign> [retrieved on 20170608]
- ANONYMOUS: "Recycold invents logistics solution for refrigerated products", 24 September 2015 (2015-09-24), XP055379519, Retrieved from the Internet <URL:https://has.nl/en/showcase/recycold-invents-logistics-solution-refrigerated-products> [retrieved on 20170608]
- ANONYMOUS: "Food", 24 September 2015 (2015-09-24), XP055379516, Retrieved from the Internet <URL:https://has.nl/en/taxonomy/term/28/showcases> [retrieved on 20170608]
- MUSEUMKWARTIER B|A|S RESEARCH ET AL: "NIEUW LEVEN IN VENLOOS", 15 January 2016 (2016-01-15), XP055379466, Retrieved from the Internet <URL:http://www.ondernemendvenlo.nl/_asset/_public/magazine-OV2016-1-lowres.pdf> [retrieved on 20170608]
- ANONYMOUS: "Recycold - Home | Facebook", 29 October 2015 (2015-10-29), XP055380069, Retrieved from the Internet <URL:https://www.facebook.com/recycoldpackaging/photos/a.1536808829944432.1073741829.1466658206959495/1536808679944447/?type=3&theater> [retrieved on 20170609]

## Description

### FIELD OF THE INVENTION

The present invention relates to a cooling element comprising a cooling material embodied as a gel, which material is enclosed in a packaging material, said packaging material comprising a laminate of a single of paper and a layer of polyethylene. The invention also relates to a method of manufacturing such cooling element as well as a box-shaped article comprising such cooling element. The cooling element should be suitable for single use and should contain measures which make it possible that the element can be recycled or degraded in an environmentally friendly manner.

### BACKGROUND OF THE INVENTION

Due to the increase of online ordering of for example food and medicines, there is a growing need for cooling elements for single use. Using such cooling elements allow for sending of cooled products such as food or medicines by transport means which lacks cooling possibilities, like by regular mail. Cooling elements for single use according to the state of the art, including so-called 'gel packs', comprise materials which are not recyclable or biologically degradable. This concerns both the cooling material and the packaging material.

Biologically degradable materials according to the state of the art which in principle could be applied in the packaging material (i.e. the housing in which the cooling material is comprised) are insufficiently stable in water and are therefore not suitable for application as a moisture barrier. It therefore follows that a packaging material of this type cannot be used in combination with cooling means in liquid or gel form.

A cooling element of the type described in the opening paragraph is known as such. In particular the patent document with publication number US 3,545,230 discloses a flexible cooling device comprised of an insoluble hydrophilic gel, which when frozen can be molded to various geometric shapes and retain that particular configuration. The gel is usually prepared in the form of a continuous reinforced tape which can then be cut in pieces and piled in multiple layers. These layers are encompassed by a packaging material to form a flexible cooling device. The described device is enclosed within a flexible packaging material and has a relatively high latent heat of fusion. In case that the element is intended for single use, a disposable product or reuse, the packaging material can be a laminate of paper on the outside and plastic such as polyethylene on the inside. A metal foil can be part of the laminate to prevent the cooling element from drying out.

The known cooling element shows several disadvantages. First, the cooling material comprises extra means to reinforce the layers of cooling material. Such a design adds to the costs of the cooling element. Moreover it remains unclear which type of paper must be used in cooling elements suitable for single use, disposal or reuse. This holds especially as many types of paper material grades are available to the market, especially in the area of recycled paper types.

JP 2010 240193 A discloses a sealant layer of a laminated exterior bag A which is formed of a laminate film that has impact resistance, pinhole resistance, and gas barrier resistance in low density polyethylene. In addition, the sealant layer 1 is biodegradable, and the front base material layer 3 may also be formed by sticking polyethylene to nonwoven fabrics.

Marwin Dekkers, "#dutchtopdesign | Facebook", (20160425), URL: https://www.facebook.com/hashtag/dutchtopdesign, (20170608), XP055379451 discloses the the Recycold^{™} coolpack is 100% food safe.

"Recycold invents logistics solution for refrigerated products", (20150924), URL: https://has.nl/en/showcase/recycold-invents-logistics-solution-refrigerated-products, (20170608), XP055379519 and "Food", (20150924), URL: https://has.nl/en/taxonomy/term/28/showcases, (20170608), XP055379516 disclose that the Recycold^{™} coolpack is an efficient means of transport for refrigerated products.

Museumkwartier B|a|s Research ET AL, "NIEUW LEVEN IN VENLOOS", (20160115), URL: http://www.ondernemendvenlo.nl/_asset/_public/magazine-OV2016-1-lowres.pdf, (20170608), XP055379466 discloses that the Recycold^{™} coolpack is foodsafe and more effective than a gel pack known in the art.

Anonymous, "Recycold - Home | Facebook", (20151029), URL: https://www.facebook.com/recycoldpackaging/photos/a.1536808829944432.1073741 829.1466658206959495/1536808679944447/?type=3&theater, (20170609), XP055380069 discloses a picture of a machine.

EP 0 050 948 A2 relates to heat-exchanging head pieces whereof one purpose (in their primary application) is to chill the entire hair bearing area of the scalp before, during and after a chemotherapy injection in order to minimize the quantity of chemicals that reaches the hair roots or follicles.

JPHo6 345 102 A relates to a heavy packaging paper bag and more particularly to an improved heavy paper packaging bag which is effective for preventing dew condensation.

JP 2001171 688 A relates to a two layer bag, and more particularly to a two layer bag in which an inner layer is formed of a synthetic resin film, an outer layer is formed of a so-called paper cloth having a synthetic resin cloth oriented toward an inner surface, a bottom portion of the inner layer is heat-sealed, and a bottom portion of the outer layer is closed.

JP 2000 255 589 A relates to an ammonium nitrate medicinal heavy bag which is used for packaging and carrying and storing ammonium nitrate explosive.

JP 2012 o66 59 A relates to a double bag and a method of manufacturing the same, and more particularly, to a double bag comprising a back sheet including at least one of a synthetic resin layer, a metal layer and a 1 paper layer and a front sheet including at least two of a two paper layer, a fabric layer and a coating layer, and a method of manufacturing the same.

WO 2006 073 341 A1 relates to a thin and heat-sealable paper-based packaging laminate for a packaging container for a liquid food product. The invention is especially directed on such a laminate that is cost-effective yet sufficiently strong and durable, and that can be formed and sealed into a packaging container of a simple and flexible shape. Moreover, despite the low thickness of the laminate, it should have sufficient barriers for food products, such as a light-barrier for light- sensitive food products, and an outer appearance that is acceptable from a customer and consumer point of view. The laminate is especially well adapted for containers for ultra high temperature (UHT) treated food products or aseptic food packaging containers.

JP 2009 072404 A teaches a cooling element comprising a cooling material embodied as a gel, which material is enclosed in a packaging material, said packaging material consisting of a laminate of a single layer of paper and a layer of polyethylene. The cooling material comprises a mixture of water and sodium carboxymethyl cellulose.

### SUMMARY OF THE INVENTION

It is an object of the invention to overcome or at least reduce and/or mitigate one or more of the disadvantages mentioned in the previous paragraph. In particular, the present invention aims at providing a cooling element suitable for single use, reuse and disposal in an environmentally friendly manner at relatively low costs. The cooling elements should also be of a simple and solid construction, thus showing a relatively high mechanical strength without applying additional reinforcing elements in its design. Furthermore, the various components of the cooling element should be food contact proof and, if incorporated in the human body, be relatively easily digestible. The invention also aims at providing an inventive method of manufacture such cooling element according to the invention as well as its use in a box-shaped article that includes one or more of such cooling elements.

Some of these and possible other objects of the invention are achieved with a cooling element comprising a cooling material embodied as a gel, which material is enclosed in a packaging material, said packaging material comprising a laminate of at least one layer of paper and at least one layer of polyethylene, which cooling element is characterized according to the invention in that the layer of paper includes a layer of kraft paper.

The inventor has found that a laminates of one or more layers of kraft paper in combination with one or more thin layers of polyethylene appear to provide an excellent packaging material in cooling elements known from the prior art. In this respect, it is noted that, contrary to popular believe, kraft paper (also known as 'brown packaging paper') is not a recycled paper type. Generally, recycled paper appears to be unsuitable for cooling elements of the present invention, as such paper usually contains unacceptable high amounts of printing materials (inks, etc.), so that they do not pass food contact proof requirements. Paper of 'food grade quality' is officially allowed to be in direct contact (for example as packaging paper) with food for consumers. In contrast to general visual perception, the brown kraft paper types are not recycled and therefore do not contain printing residues. Moreover, kraft paper appears to have a relatively high strength due to its long (virgin) fibers. This property of kraft paper makes it very suitable for designing cooling element having a relatively high mechanical strength in the absence of additional strength increasing measures, like extra reinforcement layers etc. Finally, the presence of the brown-colored kraft paper contributes to the perception of sustainability of the concept underlying the invented cooling element. The cooling element is free of metal-containing layers.

It has also been found that kraft paper at its own cannot sufficiently function as a moisture barrier between the cooling material and the surrounding area. In view thereof, the packaging material of the cooling element is made from kraft paper having a layer of a waterproof protection material comprising a polyolefin such as polyethylene. Latter layer, also referred to as a coating, is provided at least at the side of the paper which is in contact with the cooling material of the cooling element. It is noted that polyethylene is also named polythene, especially in the United Kingdom.

It has further been found that, from a mechanical point of view, the laminates comprising layers of kraft paper and polyethylene have a relatively high strength, even in case that their individual layers are thin. Laminates of paper and polyethylene in which recycled paper is applied are however prone to delamination and crumbling of the paper layers, especially when applied in cooling elements which are used under high moisture conditions. Various types of polyethylene can be used in packaging material, like high density (HDPE) and low density (LDPE) types. Although pure polyethylene is preferred, the polyolefin may also contain other constituents, like other polymerized olefins, as long as the polymerized ethylene remains the major component of the polyethylene. The laminates have a single layer of paper and a single layer of polyethylene, which layer has sub-layers of the same material, whereby the polyethylene layer is positioned at the inside of the cooling element. The manufacture of laminates of comprising one or more layers of paper and one or more layers of polyethylene as such is well known in the art.

The cooling element according to the invention can be recycled at least in three ways. First, the user of a box-shaped element cooled with the cooling element of the present invention can freeze the cooling element again for reuse. Secondly, the cooling element of the present invention can be added to empty beverage packs for recycling purposes. Almost all Dutch consumers do have special waste containers for f.e. beverage packs and there are at least collection points at supermarkets. Thirdly, the cooling element according to the current invention can be added to waste paper in case that waste containers or beverage pack collection points are absent.

An interesting embodiment of the present cooling element of the invention has the feature that the weight of the layer of kraft paper ranges between 20 and 150 g/m², preferably between 50 and 110 g/m², more preferably between 60 and 80 g/m², and most preferably around 70 g/m². The layer of paper having a weight (and indirectly a thickness) defined by the mentioned ranges have the advantage that they result in cooling elements with optimal properties, especially in the more and most preferred ranges. In case that the weight of the paper is lower than 20 g/m² the strength of the paper added to the packaging material may become too low. In case that the weight of the paper is higher than 110 g/m² the paper may become too rigid, causing that the cooling element in which such paper is applied becomes inflexible. Both possible disadvantages are virtually absent if the paper thickness ranges between 50 and 110 g/m², more preferably between 60 and 80 g/m², and most preferably around 70 g/m².

The invented cooling element shows the feature that the layer of polyethylene is formed as a laminate comprising a first layer of blown film extruded polyethylene and a second layer of cast film extruded polyethylene, whereby the surface of the first layer that faces away from the second layer is positioned on one of the major surfaces of the paper.

In the cooling element according to the invention, the two sub-layers of the polyethylene layer fulfill essentially two different functions. The first layer of polyethylene, being positioned between the layer of paper and the second layer of polyethylene, essentially acts as a moisture barrier layer between the cooling material and the outside world. Having a good moisture barrier can prevent desiccation (drying out) of the gel and wetting of the paper. Furthermore, blown film layers of PE appear to have a relatively high mechanical strength (film tensile modulus) and they adhere well to kraft paper.

The second layer of PE, being positioned on one of the major surfaces of the first layer and having its other major surface free, essentially acts as a sealing layer. Thus, two identically shaped layers of packaging material intended for a cooling element can be positioned in such a way that the second layers of PE are facing each other. A container can be made by sealing the circumferences of the two papers by heating them while pressing said second layers together. Cast films usually show a lower density resulting in a softer film, which are especially suitable for sealing properties. The properties of the blown extruded polyethylene films and the cast extruded polyethylene films are compared using layers of the same material and same thickness.

Still another embodiment of the cooling element according to the current invention has the feature that the total thickness of the polyethylene layer ranges between 5 and 50 micrometer, preferably between 20 and 45 micrometer, and most preferably between 30 and 35 micrometer.

When the polyethylene layer has a total thickness of less than 5 micron, its moisture barrier and sealing properties may be affected in a disadvantageous way. This may lead to cooling elements of which the cooling material may leak to the outside world, which is not desired. On the other hand, cooling elements having a package layer with a polyethylene layer with a total thickness that exceeds 50 micron may also result in sub-optimal products. Thus, such layers may become somewhat brittle, which could lead to cracks through which the cooling material may leak to the outside world. In view of these effects, a good compromise between the negative effects is achieved in case that the total thickness of the polyethylene layer is chosen between 20 and 45 micrometer, and preferably between 30 and 35 micrometer. In general, the thickness of the polyethylene layer is chosen as low as possible in order to keep the amount of plastics in the cooling device as low as possible.

The layer of polyethylene itself contains two or more sublayers of the same or highly similar material, which sublayers may be manufactured in different ways. Thus the layer exists of two sublayers of a layer of blown extruded polyethylene and a layer of cast extruded polyethylene. The thickness of the sublayers can be arranged in such a way that the properties of these layers are optimal for the use as part of the packaging material in a cooling element.

A further interesting cooling element according to the present invention has the feature that at least part of the polyethylene is produced from non-fossil sources. A for this purpose very suitable and recyclable material is for example polyethylene prepared from sugar cane, also referred to as Bio-PE. Although this material is not biologically degradable, its - renewable - production does not add to the use of fossil sources and provides therefore a sustainable solution. This feature can clearly distinguish the packaging material of the cooling elements according to the present invention from the containers of the prior art. In essence, such Bio-PE can be prepared by fermentation of sugars, for example obtained from sugar cane, into bio-ethanol, subsequent dehydration of the bio-ethanol into ethylene and the subsequent polymerization of such ethylene into polyethylene (or PE). Preferably all polyethylene of the invented cooling element is produced from Bio-PE.

According to the invention, the cooling material comprises a mixture of water and sodium carboxymethyl cellulose. The addition of a relatively small but effective amount of sodium carboxymethyl cellulose (few weight percent) to water during stirring at room temperature results in the formation of an aqueous gel. This gel can be used as a cooling material in the cooling element of the present invention. Said gel is usually solid up to temperatures of 30°C. Without further measures, it freezes at a temperature of 0°C at ambient pressure. A typical feature of sodium carboxymethyl cellulose is that it counteracts the formation of ice crystals. This property is advantageous as it promotes that during freezing and thawing of the gel the gel remains homogeneous. Such continues homogeneity of the cooling material prevents a lowering of its cooling capacity.

The use of an aqueous gel containing sodium carboxymethyl cellulose has the clear advantage that this material is suitable for human consumption and also digestible after human consumption. It is noted that this material is preferred over the comparable carboxymethyl cellulose (in a non-salt form), which is also suitable for human consumption, but which cannot be digested by humans.

Another embodiment of the special cooling element as invented has the feature that the sodium carboxymethyl cellulose is prepared by reacting alkali carboxymethyl cellulose with sodium monochloroacetate. Sodium carboxymethyl cellulose manufactured via this production route can be purified to high degrees, i.e. more than 99.5 percent. The process of manufacturing sodium carboxymethyl cellulose in this manner is referred to as the Blanose process. Using sodium carboxymethyl cellulose with high purity is desired in view of the requirement that the cooling material should be consumable and digestible. Having cooling elements with cooling materials of high purity is desired in order to fulfill the intended food contact regulations. In case of relatively large amount of impurities in the cooling material, the requirements for answering such regulations are difficult to realize.

According to the invention, the amount of the sodium carboxymethyl cellulose in the cooling material ranges between 1 and 4 weight percent, preferably between 1.5 and 3 weight percent, most preferably around 2 weight percent. Gels containing more than 1 percent of sodium carboxymethyl cellulose in the cooling material appear to answer the requirements of viscosity and processing whereas gels containing less than 1 percent of this compound may become too liquid for proper handling. Gels containing less than 4 percent of sodium carboxymethyl cellulose in the cooling material appear to show sufficient viscous, whereas cooling materials containing more than 4 percent of this compound may become too thick for proper handling. Compositions having a good viscosity and workability are found in case that the amount of sodium carboxymethyl cellulose in the cooling material ranges between 1.5 and 3 weight percent. Optimal properties are found for compositions containing 2 percent weight percent of sodium carboxymethyl cellulose. The weight percentages are calculated on the total weight of the gel, including the amount of water, sodium carboxymethyl cellulose and possible further additives.

A further embodiment of the cooling element of the invention is characterized in that the cooling material comprises an additive which decreases the melting point of the cooling material. The presence of such additive may be of use in case the optimal storage temperature of a body to be cooled by the cooling element is a number of degrees Centigrade below the melting point of the pure solvent used in the cooling material of the element. The additive may cause that the melting temperature of the cooling material is lowered, ideally to just below the storage temperature of the body to be cooled. Thus, when using an aqueous gel of a composition as described before, adding additives like ionic salts such as sodium chloride or molecules such as sugar can decrease the melting point of such aqueous gel to a desired value or value range (melting point trajectory).

The invention also relates to a method for manufacturing a cooling element. This method is characterized in that it comprises the following steps:
1. Providing packaging material comprising a laminate of a single layer of kraft paper and layer of polyethylene, wherein the layer of polyethylene is formed as a laminate comprising a first layer of blown film extruded polyethylene (3) and a second layer of cast film extruded polyethylene (4), whereby the surface of the first layer that faces away from the second layer is positioned on one of the major surfaces of the paper,
2. Positioning mutually two shaped pieces of the packaging material such that the layers of polyethylene are mutually facing,
3. Sealing by heat treatment the two shaped pieces at their circumference together, with the exception of a part suitable for filling,
4. Filling the thus obtained container with a cooling material, wherein the cooling material comprises a mixture of water and sodium carboxymethyl cellulose, and wherein the amount of the sodium carboxymethyl cellulose in the cooling material ranges between 1 and 4 weight percent, and
5. Sealing the part suitable for filling by heat treatment.

Packaging material comprising a laminate of at least one layer of kraft paper and at least one layer of polyethylene can be obtained from specialized paper industry. In general, the laminates are manufactured by gluing together two or more layers of the desired material, such as layers of kraft paper, blown film extruded polyethylene and/or of cast film extruded polyethylene. The so obtained laminates are stocked in rolls. Two pieces of preferably equal size are cut from laminate of any of said rolls and mutually positioned such that the layers of the polyethylene are facing each other. In a subsequent step, the rectangular pieces are heated around the largest part of their circumference, while the facing surfaces of polyethylene are pressed together. This causes sealing of both rectangular pieces at their circumference, thus forming a container. Only a small portion of the circumference is not sealed at this stage, which portion is used for filling the formed container with a gelled cooling material. After the container has been filled, the filling part of the circumference of the container is also sealed by means of heat.

An interesting embodiment of the method according to the present invention is characterized in that the cooling material is filled into the container at a temperature below room temperature. Filling the container with cooling material that has a temperature of for example less than 25°C, preferably less than 10°C has the advantage that the formation of condensation on the parts still to be sealed in the area of the filling part is minimized. Such condensation can cause that the subsequent sealing around the filling part becomes difficult, if possible at all, so that leakage can occur at this part of the sealing.

Another interesting embodiment of the method according to the present invention is characterized in that the shaped pieces of the packaging material have an essentially rectangular shape. It is observed that in principle pieces of any desired shape can be cut from the laminates in order to prepare cooling elements of such desired shape. In practice, rectangular shaped pieces are used in view of efficiency. It has been shown that rectangular pieces having a length which is the double of the width are efficient in their use in a cooling element of the present invention.

Still another interesting embodiment of the invented method has the feature that the container is filled with the cooling material such that the amount of remaining gas in the sealed cooling element is less than 2 vol.%, preferably less than 1 vol.%. It has been shown in practice that the presence of gas (usually air) in the cooling elements closed after sealing can cause difficulties. Especially when the cooling elements are in frozen and therefore inflexible form, solid devices can easily cause leaks in the packaging material of the cooling element at positions where gas bubbles are present. In order to overcome this problem, it is desirable to have no gas or air left in the container during the filling operation. However, overfilling the containers during the filling operation also causes difficulties with forming a seal around the filling opening of the container. Therefore, overfilling should be avoided as well. Experiments are going on to degas the solvent used in the cooling material, for example by pre-boiling the used solvent or by maintaining it under reduced pressure.

The invention finally relates to a box-shaped article comprising a body which needs being cooled as well as at least one cooling element according to the present invention. Such box-shaped article could be a food box, boxes for cooling medicines etc.

### BRIEF DESCRIPTION OF THE INVENTION

These and other aspects of the invention will be apparent from and elucidated with reference to the embodiments described hereinafter.

In the drawings:
Figure 1 shows schematically in perspective view a cooling element according to the present invention, and
Figure 2 show schematically in cross sectional view the same cooling element, seen along line A-A in Figure 1.

It is stressed that the drawing is schematic and not to scale. In the different Figures, same elements are denoted with same reference numbers.

### DETAILED DESCRIPTION OF EMBODIMENTS

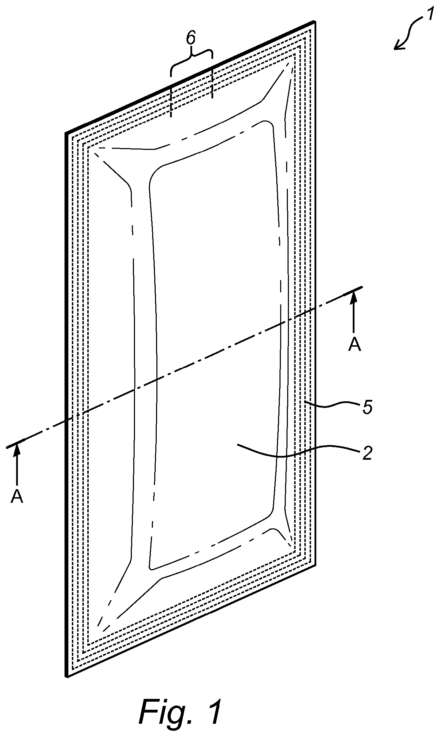

Figure 1 shows a perspective view of a cooling element 1 according to the present invention. Said cooling element comprises two substantially identical shape pieces of a laminate of packaging material which is in the present embodiment build up from three layers. The first layer 2 contains kraft paper (70 g/m²), the second layer 3 contains blown film extruded polyethylene (20 g/m²) and the third layer 4 contains cast film extruded polyethylene (27 g/m²). The total thickness of the polyethylene layer (including the two sublayers) ranges between 20 and 45 micrometer. The individual laminates of the packaging material have dimensions 24 cm in length and 12 cm in width. The cooling elements have been provided with a sealing at the circumference 5 of the laminates. Said sealing has a width of about 1 cm. The position where the container formed by the packaging material was filled is indicated by reference 6.

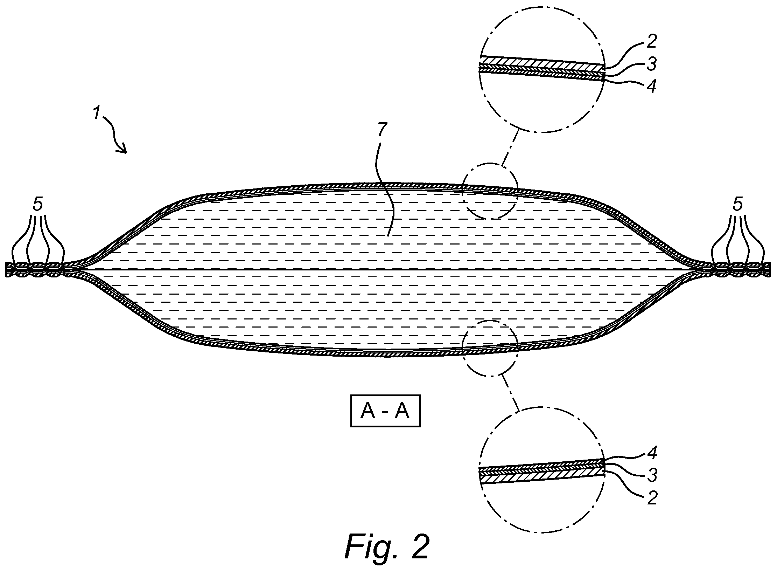

Figure 2 shows a cross section of the cooling element 1 along line A-A as shown in Figure 1. This Figure shows that a cooling material 7 is enclosed in the packaging material, of the cooling element. The three sublayers of the packaging material are indicated as well in this cross section. This Figure moreover shows that the end portions of the two laminates are sealed together via the second layer 4 of cast film extruded polyethylene, at the end portions of the shaped pieces as indicated by reference 5.

The cooling elements shown in Figures 1 and 2 have been manufactured as follows. First a cooling material was prepared. An amount of (preferably pre-boiled) water was heated to a temperature of at least 4°C and at maximum 40°C. An amount of sodium carboxymethyl cellulose (Ashland, grade de Blanose,) was gradually added during stirring until its concentration was 2 weight percent. Where necessary, the so-obtained solution was cooled to a temperature below room temperature before it was used to fill the containers of the cooling elements. A gel having a desired viscosity was formed in this way.

In a second step, pieces of a laminate having dimensions of 12 cm x 24 cm were provided. Said laminates contained of a layer of kraft paper, and two layers of polyethylene, as described above. Pairs of pieces were position such that the layers of polyethylene were facing. The edges of the circumference of the so-positioned pairs of laminates were clamped together over an area of appr. 1 cm from their outer ends by means of heating clamps. As a result, the pairs were sealed together over this area, thus forming a type of container. Only a small length at the middle of one of the small ends was not clamped together. This small length (indicated with reference 6 in Figure 1) was not sealed so that it could function as a part suitable for filling the container.

Subsequently, the (cooled) cooling material was entered in the containers via a filling needle. The containers were filled completely, while taking care that the not (yet) sealed filling part of the container was not contaminated with the cooling material, for example as a result of overflow or as a result of condensation. After the filling operation, the small length 6 which acted as filling part was also sealed by means of a heating clamp. The sealing temperature was chosen between 50°C and 90 °C. Care was taken that the containers were filled for more than 99% by volume, so that the amount of gas or air in the container was neglectable.

In a standardized comparative test, in a container with food parts which had been cooled to -2°C, four cooling elements of the above-described type (each having a weight of 500 g) were packed. The cooling elements had been cooled to -23°C for a period of 72 hours. The container with the packed cooling elements was maintained in a room with an ambient temperature of 20 °C. It appeared that the packed food parts could be maintained for 21 hours at a temperature below 5 °C. Cooling elements not according to the present invention which moreover contained silica gel as a cooling material could maintain the food parts only for 14 hours below this temperature under further identical conditions.

In an additional series of experiments it was investigated whether melting point decreasing effect of NaCl (sodium chloride) could be exploited in cooling elements according to the invention containing an aqueous gel of 2 weight percent sodium carboxymethyl cellulose. The effect of the NaCl additive on the cooling capacity of the cooling element was investigated as well. In this series of experiments, amounts of NaCl (in weight percent's) were added to the gel: 0% (comparative example A), 1% (example B), 5% (example C) and 10% (example D). It appeared that expected melting point decreases were obtained. It appeared however from the results of examples C and D that the additive caused lower cooling capacity. Example B showed a cooling capacity which was comparable to the cooling capacity of the example without added NaCl (example A).

While the invention has been illustrated and described in detail in the drawing and foregoing description, such illustration and description are to be considered illustrative or exemplary and not restrictive. The invention is not limited to the disclosed embodiments.

In the claims, the word 'comprising' does not exclude other elements or steps, and the indefinite article 'a' or 'an' does not exclude a plurality. The mere fact that certain measures are recited in mutually different claims does not indicate that a combination of these measures cannot be used to advantage. Any reference signs in the claims should not be construed as limiting the scope of these claims.

## Claims

1. Cooling element (1) comprising a cooling material embodied as a gel, which material is enclosed in a packaging material, said packaging material consisting of a laminate of a single layer of paper and a layer of polyethylene,
wherein the cooling material (7) comprises a mixture of water and sodium carboxymethyl cellulose,
wherein the amount of the sodium carboxymethyl cellulose in the cooling material (7) ranges between 1 and 4 weight percent; **characterized in that**
the layer of paper includes a layer of kraft paper (2); and
the layer of polyethylene is formed as a laminate comprising a first layer of blown film extruded polyethylene (3) and a second layer of cast film extruded polyethylene (4),
whereby the surface of the first layer that faces away from the second layer is positioned on one of the major surfaces of the paper.

2. Cooling element (1) according to claim 1, **characterized in that** the weight of the layer of kraft paper ranges between 20 and 150 g/m², preferably between 50 and 110 g/m², more preferably between 60 and 80 g/m², and most preferably around 70 g/m².

3. Cooling element (1) according to any of the previous claims, **characterized in that** the total thickness of the polyethylene layer ranges between 5 and 50 micrometer, preferably between 20 and 45 micrometer, and most preferably between 30 and 35 micrometer.

4. Cooling element (1) according to any of the previous claims, **characterized in that** at least part of the polyethylene is produced from non-fossil sources.

5. Cooling element (1) according to claim 1, **characterized in that** the sodium carboxymethyl cellulose is prepared by reacting alkali carboxymethyl cellulose with sodium monochloroacetate.

6. Cooling element according to any of the previous claims, **characterized in that** the amount of the sodium carboxymethyl cellulose in the cooling material ranges between 1.5 and 3 weight percent, most preferably around 2 weight percent.

7. Cooling element (1) according to any of the previous claims, **characterized in that** the cooling material (7) comprises an additive which decreases the melting point of the cooling material (7).

8. Method for manufacturing a cooling element (1), **characterized in that** it comprises the following steps:
1. Providing packaging material comprising a laminate of a single layer of kraft paper and a layer of polyethylene, wherein the layer of polyethylene is formed as a laminate comprising a first layer of blown film extruded polyethylene (3) and a second layer of cast film extruded polyethylene (4), whereby the surface of the first layer that faces away from the second layer is positioned on one of the major surfaces of the paper,
2. Positioning mutually two shaped pieces of the packaging material such that the layers of polyethylene are mutually facing,
3. Sealing by heat treatment the two shaped pieces at their circumference together, with the exception of a part suitable for filling,
4. Filling the thus obtained container with a cooling material,
wherein the cooling material comprises a mixture of water and sodium carboxymethyl cellulose, and
wherein the amount of the sodium carboxymethyl cellulose in the cooling material ranges between 1 and 4 weight percent, and
5. Sealing the part suitable for filling by heat treatment.

9. Method according to claim 8, **characterized in that** the cooling material (7) is filled into the container at a temperature below room temperature.

10. Method according to any of claims 8 and 9, **characterized in that** the shaped pieces of the packaging material have an essentially rectangular shape.

11. Method according to any of the claims 8 to 10, **characterized in that** the container is filled with the cooling material (7) such that the amount of remaining gas in the sealed cooling element is less than 2 vol.%, preferably less than 1 vol.%.

12. Box-shaped article comprising a body which needs being cooled as well as a cooling element (1) according to any of the previous claims 1 - 7.

## Patentansprüche

1. Kühlelement (1), umfassend ein Kühlmaterial, das als Gel ausgeführt ist, wobei das Material in einem Verpackungsmaterial eingeschlossen ist, wobei das Verpackungsmaterial aus einem Laminat aus einer einzelnen Papierschicht und einer Polyethylenschicht besteht,
wobei das Kühlmaterial (7) eine Mischung aus Wasser und Natriumcarboxymethylcellulose umfasst,
wobei die Menge der Natriumcarboxymethylcellulose in dem Kühlmaterial (7) zwischen 1 und 4 Gewichtsprozent liegt; **dadurch gekennzeichnet, dass** die Papierschicht eine Kraftpapierschicht (2) umfasst; und
die Polyethylenschicht als ein Laminat gebildet ist, das eine erste Schicht aus blasfolienextrudiertem Polyethylen (3) und eine zweite Schicht aus gießfolienextrudiertem Polyethylen (4) umfasst, wobei die Oberfläche der ersten Schicht, die von der zweiten Schicht abgewandt ist, auf einer der Hauptoberflächen des Papiers positioniert ist.

2. Kühlelement (1) nach Anspruch 1, **dadurch gekennzeichnet, dass** das Gewicht der Kraftpapierschicht zwischen 20 und 150 g/m², vorzugsweise zwischen 50 und 110 g/m², mehr bevorzugt zwischen 60 und 80 g/m² und am meisten bevorzugt etwa 70 g/m² liegt.

3. Kühlelement (1) nach einem der vorhergehenden Ansprüche, **dadurch gekennzeichnet, dass** die Gesamtdicke der Polyethylenschicht zwischen 5 und 50 Mikrometer, vorzugsweise zwischen 20 und 45 Mikrometer und am meisten bevorzugt zwischen 30 und 35 Mikrometer liegt.

4. Kühlelement (1) nach einem der vorhergehenden Ansprüche, **dadurch gekennzeichnet, dass** mindestens ein Teil des Polyethylens aus nicht fossilen Quellen hergestellt ist.

5. Kühlelement (1) nach Anspruch 1, **dadurch gekennzeichnet, dass** die Carboxymethylcellulose durch Umsetzen von Alkalicarboxymethylcellulose mit Natriummonochloracetat hergestellt ist.

6. Kühlelement nach einem der vorhergehenden Ansprüche, **dadurch gekennzeichnet, dass** die Menge der Natriumcarboxymethylcellulose in dem Kühlmaterial zwischen 1,5 und 3 Gewichtsprozent, am meisten bevorzugt etwa 2 Gewichtsprozent liegt.

7. Kühlelement (1) nach einem der vorhergehenden Ansprüche, **dadurch gekennzeichnet, dass** das Kühlmaterial (7) einen Zusatzstoff umfasst, der den Schmelzpunkt des Kühlmaterials (7) verringert.

8. Verfahren zur Herstellung eines Kühlelements (1), **dadurch gekennzeichnet, dass** es die folgenden Schritte umfasst:
1. Bereitstellen von Verpackungsmaterial, umfassend ein Laminat aus einer einzelnen Kraftpapierschicht und einer Polyethylenschicht, wobei die Polyethylenschicht als ein Laminat gebildet ist, das eine erste Schicht aus blasfolienextrudiertem Polyethylen (3) und eine zweite Schicht aus gießfolienextrudiertem Polyethylen (4) umfasst, wobei die Oberfläche der ersten Schicht, die von der zweiten Schicht abgewandt ist, auf einer der Hauptoberflächen des Papiers positioniert ist,
2. gegenseitiges Positionieren von zwei Formstücken des Verpackungsmaterials, so dass die Polyethylenschichten einander zugewandt sind,
3. Versiegeln der zwei Formstücke an ihrem Umfang miteinander durch Wärmebehandlung, mit Ausnahme eines zum Füllen geeigneten Teils,
4. Füllen des so erhaltenen Behälters mit einem Kühlmaterial,
wobei das Kühlmaterial eine Mischung aus Wasser und Natriumcarboxymethylcellulose umfasst, und
wobei die Menge der Natriumcarboxymethylcellulose in dem Kühlmaterial zwischen 1 und 4 Gewichtsprozent liegt, und
5. Versiegeln des zum Füllen geeigneten Teils durch Wärmebehandlung.

9. Verfahren nach Anspruch 8, **dadurch gekennzeichnet, dass** das Kühlmaterial (7) bei einer Temperatur unter Raumtemperatur in den Behälter gefüllt wird.

10. Verfahren nach einem der Ansprüche 8 und 9, **dadurch gekennzeichnet, dass** die Formstücke des Verpackungsmaterials eine im Wesentlichen rechteckige Form aufweisen.

11. Verfahren nach einem der Ansprüche 8 bis 10, **dadurch gekennzeichnet, dass** der Behälter mit dem Kühlmaterial (7) gefüllt wird, so dass die Menge an verbleibendem Gas in dem versiegelten Kühlelement weniger als 2 Vol.-%, vorzugsweise weniger als 1 Vol.-% beträgt.

12. Kastenförmiger Artikel, umfassend einen Körper, der gekühlt werden muss, sowie
Kühlelement (1) nach einem der vorhergehenden Ansprüche 1 bis 7.

## Revendications

1. Elément refroidissant (1) comprenant un matériau refroidissant incorporé sous forme d'un gel, matériau qui est enfermé dans un matériau d'emballage, ledit matériau d'emballage étant constitué d'un stratifié d'une unique couche de papier et d'une couche de polyéthylène,
dans lequel le matériau refroidissant (7) comprend un mélange d'eau et de carboxyméthycellulose de sodium,
dans lequel la teneur de la carboxyméthylcellulose de sodium dans le matériau refroidissant (7) est comprise entre 1 et 4 pourcents en poids ; **caractérisé en ce que** :
la couche de papier comprend une couche de papier kraft (2) ; et
la couche de polyéthylène est formée sous la forme d'un stratifié comprenant une première couche de polyéthylène extrudé en film soufflé (3) et une seconde couche de polyéthylène extrudé en film coulé (4), de telle façon que la surface de la première couche qui est tournée en éloignement de la seconde couche soit positionnée sur l'une des surfaces principales du papier.

2. Elément refroidissant (1) selon la revendication 1, **caractérisé en ce que** le poids de la couche de papier kraft est compris entre 20 et 150 g/m², de préférence entre 50 et 110 g/m², plus préférablement entre 60 et 80 g/m², et le plus préférentiellement autour de 70 g/m².

3. Elément refroidissant (1) selon l'une des revendications précédentes, **caractérisé en ce que** l'épaisseur totale de la couche de polyéthylène est comprise entre 5 et 50 micromètres, de préférence entre 20 et 45 micromètres, et le plus préférentiellement entre 30 et 35 micromètres.

4. Elément refroidissant (1) selon l'une des revendications précédentes, **caractérisé en ce qu'**au moins une partie du polyéthylène est produit à partir de sources non fossiles.

5. Elément refroidissant (1) selon la revendication 1, **caractérisé en ce que** la carboxyméthylcellulose de sodium est préparée en faisant réagir une carboxyméthylcellulose alcaline avec du monochloroacétate de sodium.

6. Elément refroidissant (1) selon l'une des revendications précédentes, **caractérisé en ce que** la teneur de la carboxyméthylcellulose de sodium dans le matériau refroidissant est comprise entre 1,5 et 3 % en poids, plus préférentiellement autour de 2 % en poids.

7. Elément refroidissant (1) selon l'une des revendications précédentes, **caractérisé en ce que** le matériau refroidissant (7) comprend un additif qui abaisse le point de fusion du matériau refroidissant (7).

8. Procédé de fabrication d'un élément refroidissant (1), **caractérisé en ce qu'**il comprend les étapes suivantes :
1. obtention d'un matériau d'emballage comprenant un stratifié d'une unique couche de papier kraft et d'une couche de polyéthylène, la couche de polyéthylène étant formée sous forme d'un stratifié comprenant une première couche de polyéthylène extrudé en film soufflé (3) et d'une seconde couche de polyéthylène extrudé en film coulé (4), de telle façon que la surface de la première couche qui est tournée en éloignement de la seconde couche soit positionnée sur l'une des surfaces principales du papier,
2. positionnement l'une par rapport à l'autre de deux pièces conformées du matériau d'emballage de telle manière que les couches de polyéthylène soient tournées l'une vers l'autre,
3. scellement par traitement thermique des deux pièces conformées ensemble à leur circonférence, à l'exception d'une partie permettant le remplissage,
4. remplissage du conteneur ainsi obtenu par un matériau de refroidissement,
le matériau de refroidissement comprenant un mélange d'eau et de carboxyméthylcellulose de sodium, et
la teneur de la carboxyméthylcellulose de sodium dans le matériau refroidissant étant comprise entre 1 et 4 % en poids, et
5. scellement par traitement thermique de la partie permettant le remplissage.

9. Procédé selon la revendication 8, **caractérisé en ce que** le matériau refroidissant (7) est rempli dans le conteneur à une température inférieure à la température ambiante.

10. Procédé selon l'une des revendications 8 et 9, **caractérisé en ce que** les pièces conformées du matériau d'emballage présentent une forme essentiellement rectangulaire.

11. Procédé selon l'une des revendications 8 à 10, **caractérisé en ce que** le conteneur est rempli avec le matériau refroidissant (7) de telle manière que la quantité de gaz subsistant dans l'élément refroidissant scellé soit inférieure à 2 % en volume, de préférence inférieure à 1 % en volume.

12. Article en forme de boîte comprenant un corps nécessitant d'être refroidi, ainsi qu'un élément refroidissant (1) selon l'une des revendications 1 à 7 précédentes.
